# EUROPEAN PATENT SPECIFICATION

(11) **EP 1 979 412 B1**
(45) Date of publication and mention of the grant of the patent: **03.06.2009**
(21) Application number: 06845265.5
(22) Date of filing: 12.12.2006
(51) Int. Cl.: C08L 67/00, C08L 67/04

(54) **BIOABSORBABLE POLYMER COMPOSITIONS EXHIBITING ENHANCED CRYSTALLIZATION AND HYDROLYSIS RATES**
BIOABSORBIERBARE POLYMERZUSAMMENSETZUNGEN MIT ERHÖHTEN KRISTALLISATIONS- UND HYDROLYSERATEN
COMPOSITIONS POLYMERES BIOABSORBABLES A TAUX DE CRISTALLISATION ET D'HYDROLYSE AMELIORES

(30) Priority: 28.12.2005 US 320029
(43) Date of publication of application: 15.10.2008
(73) Proprietor: Ethicon, Inc, Somerville, NJ 08876-0151 (US)
(72) Inventor: ANDJELIC, Sasa, Nanuet, New York 10964 (US); FITZ, Benjamin D., Brooklyn, New York 11225 (US)
(74) Representative: James, Anthony Christopher W.P.
(86) International application number: PCT/US2006/047339
(87) International publication number: WO 2007/078718

(56) References cited:
- WO-A-20/04043432
- US-A1- 2004 022 859
- US-A1- 2005 124 941
- VON RECUM H A ET AL: "Degradation of polydispersed poly(l-lactic acid) to modulate lactic acid release" BIOMATERIALS, ELSEVIER SCIENCE PUBLISHERS BV., BARKING, GB, vol. 16, no. 6, 1995, pages 441-447, XP004032951 ISSN: 0142-9612 cited in the application

## Description

### FIELD

This invention relates to absorbable polymer compositions and, more particularly, to bioabsorbable polymer compositions having a bimodal molecular weight distribution, to medical devices produced therefrom and to methods of making bioabsorbable polymer compositions.

### BACKGROUND

In polymeric crystals, polymer chains are arranged in a two-dimensional pattern. Due to statistical and mechanical requirements, a complete polymer chain cannot form a single straight stem, the straight stems being limited to a certain length depending on the crystallization temperature. As a result thereof, the stems fold and reenter into a lattice. This reentry can be adjacent to the previous stem or at a random lattice point. The perfectly ordered portion of a polymer is crystalline and the folded surface is amorphous. As such, polymers are semi-crystalline. The crystalline portion may occur either in isolation or as an aggregate with other similar crystals leading to the formation of mats or bundles or spherulites.

The first step in the formation of spherulites, wherein a straight stem of a polymer chain called a nucleus forms from a random coil, is called nucleation. The rest of the process that includes lamellae growth and spherulite formation is cumulatively called crystal growth. In general, single crystals take the form of thin lamellae that are relatively large in two dimensions and bounded in the third dimension by the folds. Typically all the lamellae within one spherulite originate from a single point. As the spherulite grows, the lamellae get farther and farther apart. When the distance between two lamellae reaches a critical value, they tend to branch. Since the growth process is isotropic, the spherulites have a circular shape in two dimensions and a spherical shape in three dimensions for solidification in a uniform thermal field.

A certain degree of crystallinity is often desired during injection molding or extrusion operations due to the higher thermal and mechanical stability associated therewith. If the crystallization rate is slow or uneven, the resultant product properties may have a wide variation in morphology, creating a potential for lines of imperfection that may lead to material failure and result in lower production capacity and reduced quality of the final product.

Absorbable polymers are known to be generally slow crystallizing materials. As is well known to those skilled in the art, poly(L-lactic acid) (PLLA) belongs to the group of very slow crystallizable polyesters. High molecular weight PLLA crystallizes with even more difficulty, due to the reduced mobility of its highly entangled macromolecules. The crystallinity of different molecular weight PLLA polymers (18,000, 31,000, 156,000 and 425,000 g/mol) has been studied by calorimetric methods (see: Clinical Materials, 1991, 8(1-2), 111. As demonstrated by that study, during cooling from the melt (rate = -0.5°C/min), only the lower molecular weight polymers were able to develop any measurable crystallinity.

In order to increase the rate of crystallization of a polymer, one must increase either the steady-state concentration of nuclei in the polymer matrix, or increase the rate of crystal growth. In general; an increase in nucleation density can be readily accomplished by adding nucleating agents that are either physical (inactive) or chemical (active) in nature. An introduction of foreign particles can also serve as a nucleation agent: For example, with regard to the absorbable polymers used by the medical industry, such agents can include starch, sucrose, lactose, fine polymer particles of polyglycolide and copolymers of glycolide and lactide, which may be used during manufacturing of surgical fasteners or during subsequent fiber processing. Other ways to increase the nucleation rate without the addition of foreign-based materials include copolymerization with a stiffer, highly crystallizable component, preserving nucleating seeds of a faster crystallizing component during melt manufacturing steps, stress induced nucleation, the use of magnetic field strength or sonic-based energy, as used by the pharmaceutical industry, and the use of specific ratios of mono-to bi-functional initiators in the ring-opening polymerization of glycolide-containing absorbable copolyesters.

With regard to the absorbable polymers having utility in the area of wound management, improved hydrolysis characteristics are often desired to reduce the incidence of infection and increase patient comfort. Improved hydrolysis characteristics are also desired in the area of drug delivery to enhance drug release.

In order to control or increase the bioabsorption/hydrolysis rate of absorbable polymers, several approaches have been proposed. These include exposure to high-energy radiation, such as gamma rays or electron beam radiation treatment under an oxygen atmosphere, blending or copolymerizing the absorbable slow degrading polymer with a faster absorbing material, use of a pore-forming component, varying the pH value of materials having pH sensitive groups and addition of monomers or oligomers to the polymer matrix.

It has been proposed in U.S. Patent No. 5,539,076 that bimodal molecular weight distributions may be employed for polyolefins to enhance polymer processing, reduce the tendency of die-lip polymer buildup and smoking in on-line operations. Moreover, the crystallization behavior of various binary compositions has been reported for linear polyethylene blends in Polymer, 1998, 29(6), 1045. This study suggests that the two fractions of a binary linear polyethylene blend crystallize separately and independently at moderate and high temperatures and partially co-crystallize at lower temperatures. Similarly, Cheng and Wunderlich, in J. Polym. Sci. Polym. Phys., 1986, 24, 595 and J. Polym. Sci. Polym. Phys., 1991, 29, 515, reported on their crystallization kinetic studies of fractions of poly(ethylene oxides) between 3,500 and 100,000 Mw and their binary mixtures from the melt. These studies suggested that mixed-crystal formation at low crystallization temperatures occurred, with increasing segregation at higher temperatures, despite the higher deposition probabilities of the low molecular weight component.

Von Recum, H. A, Cleek, R. L., Eskint, S. G., and Mikos, A. G., in Biomaterials 18, 1995, 441-447, suggested that modulating lactic acid release during *in vivo* degradation of PLLA implants, by adjusting the polymer polydispersity, was feasible. In their work, polydispersed PLLA membranes comprised of blends of monodispersed PLLA of weight average molecular weight of 82500 and 7600 Daltons were fabricated to investigate the effect of polydispersity on degradation characteristics. The PLLA blends exhibited large spherulites of high molecular weight chains embedded in a low molecular weight matrix. During degradation in a phosphate buffer, the release rate of lactic acid increased as the percentage of the low molecular weight component in the blend was increased. For low molecular weight compositions larger than 50%, voids were created in the degrading blends due to the degradation of low molecular weight chains and the concurrent dissolution of lactic acid, and also the release of undegraded particles of high molecular weight.

Despite these advances in the art, there is still a need for improved absorbable polymers having increased crystallization and/or hydrolysis rates. Thus, it would-be desirable to provide advanced absorbable polymers having increased crystallization and/or hydrolysis rates and methods for their production.

### SUMMARY

Disclosed herein are compositions and methods of enhancing the crystallization and/or hydrolysis rates for absorbable materials. Also disclosed are methods of preparation of absorbable polymer compositions, the compositions so prepared possessing significantly higher crystallization kinetics and/or hydrolysis rates, and devices produced from such compositions. More specifically disclosed herein are absorbable polymeric blend compositions, processes of making the absorbable polymeric blend compositions and medical devices produced from such absorbable polymeric blend compositions.

In one aspect, provided is a bimodal polymer composition, comprising: a first amount of a bioabsorbable polymer having a first molecular weight distribution; and a second amount of the bioabsorbable polymer having a second molecular weight distribution having a weight average molecular weight between about 20,000 to about 50,000 Daltons, the weight average molecular weight ratio of the first molecular weight distribution to the second molecular weight distribution being at least about two to one; wherein a substantially homogeneous blend of the first and second amounts of the bioabsorbable polymer is formed in a ratio of between about 50/50 to about 95/5 weight/weight percent.

In another aspect, provided is a medical device produced from a process comprising the step of injection molding or extruding the medical device from a bimodal polymer composition or the step of subjecting a medical device made from said bimodal polymer composition to a heat treatment step, the bimodal polymer composition comprising: a first amount of a bioabsorbable polymer polymerized so as to have a first molecular weight distribution; and a second amount of the bioabsorbable polymer polymerized so as to have a second molecular weight distribution having a weight average molecular weight between about 20,000 to about 50,000 Daltons, the weight average molecular weight ratio of the first molecular weight distribution to the second molecular weight distribution being at least about two to one; wherein a substantially homogeneous blend of the first and second amounts of the bioabsorbable polymer is formed in a ratio of between about 50/50 to about 95/5 weight/weight percent.

In yet another aspect, provided is a medical device produced from a process comprising the step of injection molding or extruding the medical device from a bimodal polymer composition or the step of subject a medical device made from said bimodal polymer composition to a heat treatment step; said bimodal polymer composition comprising: a first amount of a poly(L-lactide) polymer having a first molecular weight distribution between about 100,000 to about 2,000,000 Daltons; and a second amount of a poly(L-lactide) polymer having a second molecular weight distribution having a weight average molecular weight between about 20,000 to about 50,000 Daltons, the weight average molecular weight ratio of said first molecular weight distribution to said second molecular weight distribution is at least about two to one; wherein a substantially homogeneous blend of said first and second amounts of said bioabsorbable polymer is formed in a ratio of between about 60/40 to 80/20 weight/weight percent; over a temperature range of between about 85°C to about 150°C.

In still yet another aspect, provided is a medical device produced from a process comprising the step of injection molding or extruding the medical device from a bimodal polymer composition or the step of subject a medical device made from said bimodal polymer composition to a heat treatment step; said bimodal polymer composition comprising: a first amount of a poly(dioxanone) polymer having a first molecular weight distribution between about 50,000 to about 100,000 Daltons; and a second amount of a poly(dioxanone) polymer having a second molecular weight distribution having a weight average molecular weight between about 20,000 to about 50,000 Daltons, the weight average molecular weight ratio of said first molecular weight distribution to said second molecular weight distribution is at least about two to one; wherein a substantially homogeneous blend of said first and second amounts of said bioabsorbable polymer is formed in a ratio of between about 60/40 to 95/5 weight/weight percent; over a temperature range of between about 40°C to about 80°C.

In a further aspect, provided is a method of making a medical device, comprising the step of injection molding or extruding the medical device from a bimodal polymer composition or the step of subjecting a medical device made from the bimodal polymer composition to a heat treatment step, the polymer composition, comprising: a first amount of a bioabsorbable polymer polymerized so as to have a first molecular weight distribution; a second amount of the bioabsorbable polymer polymerized so as to have a second molecular weight distribution having a weight average molecular weight between about 20,000 to about 50,000 Daltons, the weight average molecular weight ratio of the first molecular weight distribution to the second molecular weight distribution being at least about two to one; wherein a substantially homogeneous blend of said first and second amounts of said bioabsorbable polymer is formed in a ratio of between about 60/40 to 95/5 weight/weight percent.

In a still further aspect, provided is a method of making a medical device, comprising the step of injection molding or extruding said medical device from a bimodal polymer composition or the step of subject a medical device made from said bimodal polymer composition to a heat treatment step; the bimodal polymer composition comprising: a first amount of a poly(L-lactide) polymer having a first molecular weight distribution between about 100,000 to about 1,000,000 Daltons; and second amount of a poly(L-lactide) polymer having a second molecular weight distribution having a weight average molecular weight between about 20,000 to about 50,000 Daltons, the weight average molecular weight ratio of said first molecular weight distribution to said second molecular weight distribution is at least about two to one; wherein a substantially homogeneous blend of said first and second amounts of said bioabsorbable polymer is formed in a ratio of between about 60/40 to 80/20 weight/weight percent; over a temperature range of between about 85°C to about 150°C.

In a yet still further aspect, provided is a method of making a medical device, comprising the step of injection molding or extruding said medical device from a bimodal polymer composition or the step of subject a medical device made from said bimodal polymer composition to a heat treatment step; the bimodal polymer composition comprising: a first amount of a poly(dioxanone) polymer having a first molecular weight distribution between about 50,000 to about 100,000 Daltons; and a second amount of a poly(dioxanone) polymer having a second molecular weight distribution having a weight average molecular weight between about 20,000 to about 50,000 Daltons, the weight average molecular weight ratio of said first molecular weight distribution to said second molecular weight distribution is at least about two to one; wherein a substantially homogeneous blend of said first and second amounts of said bioabsorbable polymer is formed in a ratio of between about 60/40 to-95/5 weight/weight percent; over a temperature range of between about 40°C to about 80°C.

### BRIEF DESCRIPTION OF THE DRAWINGS

The invention will now be described in more detail with reference to the forms herein disclosed, given only by way of example, and with reference to the accompanying drawings, in which:

FIG.1 presents a plot of crystallization rate (expressed as t_{1/2}) as a function of crystallization temperature for several PLLA homopolymers, as determined by differential scanning calorimetry (DSC) measurements;

FIG. 2 presents GPC molecular weight distribution curves for several PLLA homopolymers;

FIG. 3 presents DSC crystallization kinetics for a Test Sample 1 polymer before and after purification, as a function of crystallization temperature. Data for PLLA homopolymer 700k are added for comparison;

FIG. 4 presents DSC crystallization kinetics for two PLLA homopolymers (300k and 50k), and a variety of blends thereof, as a function of crystallization temperature;

FIG. 5 presents DSC crystallization kinetics for two PDS homopolymers (80k and 24k) and three blend compositions thereof, as a function of crystallization temperature;

FIG. 6 presents DSC isothermal crystallization kinetics for an 80k PDS homopolymer and two blend compositions, as a function of crystallization temperature;

FIG. 7 presents DSC non-isothermal crystallization kinetics for an 80k PDS homopolymer and two blend compositions, during the cooling from the melt at a constant cooling rate of 10°C/min;

FIG.8 presents hydrolysis profiles for two PLLA homopolymers (300k and 50k) and a 70/30 blend thereof;

FIG. 9 presents hydrolysis profiles of two PDS homopolymers (90k and 5k) and their 70/30 blend for comparative purposes; and

FIG. 10 presents hydrolysis profiles of a PDS II fiber monofilament, 80k and 24k PDS homopolymers, and various blends, including 95/5, 90/10, 80/20, and 70/30 compositions.

### DETAILED DESCRIPTION

The compositions described herein provide significantly higher crystallization rates over the crystallization rates of the individual components. Additionally, the compositions described herein provide significantly higher rates of hydrolysis over the rates of hydrolysis of the individual components of those compositions.

- The absorbable polymer compositions comprise physical- blends of regular-to-high molecular weight polymer with a lower molecular weight counterpart of the same material as a minor component. The polymer blends are directed to semi-crystalline materials. The semi-crystalline polymers have enhanced processability during extrusion and/or injection molding operations, due to synergistically faster crystallization kinetics. Binary blends of semi-crystalline polymers described herein have synergistically higher hydrolysis rates compared to individual components, and may provide more uniform hydrolysis characteristics throughout the polymer matrix.

The presence of lower molecular weight polymer does not affect the nucleation density of the original material, but greatly increases the growth rate of polymer spherulites. When compositions are produced from the blend of high and low molecular weight polymers disclosed herein, the rate of crystallization may be at least about 2 times faster than the rate of crystallization over an absorbable polymer made by a substantially similar polymerization process utilizing individual components. Thus, the compositions disclosed herein provide increased crystallization and/or hydrolysis rates as compared to conventional processing, as taken under the same or similar measurement conditions or techniques.

Increased crystallization, as used herein, relates- to the improvement in the crystallization properties of a polymer, yielding a polymer that crystallizes at a faster rate. Crystallizing at a faster rate has advantages when melt processing the polymers disclosed herein. This is especially true when fabricating medical devices using an injection molding or fiber extrusion process. Rapid crystallization is particularly advantageous when injection molding articles from resins with low glass transition temperatures, since dimensional stability is usually achieved by crystallization. In the absence of crystallization, injection molded parts made from polymers possessing low glass transition temperatures also frequently display distortion and deformation upon removal from the mold, as they are not able to withstand the forces exerted, however mild, during the removal process.

As those skilled in the art will readily understand, as articles crystallize faster, cycle times may be decreased. Not only are there potential economic advantages resulting from the attendant decreased production costs, but faster cycle times also reduce the time the polymer resides in the machine at elevated temperatures. This reduces the amount of degradation that may occur, further improving part quality. The amount of crystallinity needed in the part prior to ejection from the mold depends on the glass transition temperature of the resin as well as the molecular weight of the resin. The lower the glass transition temperature, the higher the level of crystallinity required. It has been found that it is advantageous to have a crystallinity level of at least 10% for some synthetic absorbable polymers possessing low glass transition temperatures. In the case of fibers of higher molecular orientation, the level of crystallinity required is correspondingly higher; at least about 15% and desirably greater than about 25% may be necessary to provide dimensional stability.

Polymers contemplated for use herein include the class of polymers known as bioabsorbable polymers. These include poly(lactide), including L (-), D (+), meso and racemic lactide form, poly(glycolide), poly(dioxanone), poly(ε-caprolactone), poly(hydroxybutyrate), poly(β-hydroxybutyrate), poly(hydroxyvalerate), poly(tetramethylene carbonate), and poly(amino acids) and copolymers and terpolymers thereof. Also having utility herein are the materials selected from the group consisting of polyester amides, poly(phosphoresters)s, polyphosphazenes, poly(orthoester)s, poly(anhydride)s, anionic carbohydrate polymers, polysaccharides, poly(hydroxybutyric acid)s, polyacetals, poly(dl-lactide-co-glycolide)s, poly(l-lactide-co- glycolide)s, poly(alkylene diglycolate)s, poly(oxaester)s, poly(oxaamide)s, sulfonated aliphatic-aromatic copolyether esters, glyceride and dihydroxyacetone polymers.

In a first disclosed form, the polymer blends described herein include homogenous physical mixtures of the same polymers having two distinct molecular weight distributions, wherein a weight average molecular weight ratio of the first molecular weight distribution to the second molecular weight distribution is at least or greater than about two to one. Preferably, this ratio may be about three to one, more preferably in the range of about four to six to one. The amount of the first and the second molecular distributions is preferably in ratios to each other of between about 50/50 to about 95/5 (weight/weight) percent. More preferably, this ratio is between 70/30 and 95/5, respectively.

As indicated above, the polymeric blends disclosed herein are two component blends of a bioabsorbable polymeric material, each component selected on the basis of its weight average molecular weight distribution. The first component is selected to possess a weight average molecular weight between about 50,000 to about 2,000,000 Daltons. The second component is selected to possess a weight average molecular weight between about 20,000 to about 50,000 Daltons.

In another form, the composition comprises a two component poly(L-lactide) blend having a first component of a weight average molecular weight between about 50,000 to about 1,000,000 Daltons, preferably between about 100,000 to about 500,000 Daltons, and a second component of a weight average molecular weight between about 20,000 to about 50,000 Daltons. The first and second polymer components are blended in a ratio between about 60/40 to 80/20 (weight/weight) percent, respectively. The rate of crystallization of the composition is at least about two times or greater than the rate of crystallization exhibited by either the first or second polymer component alone, when evaluated using isothermal crystallization over a temperature range of between about 85°C to about 150°C. The composition is capable of crystallizing in the range of between about 140°C to about 150°C, as verified by calorimetric measurements. Similarly, the rate of hydrolysis of the composition, measured in distilled water at a constant pH value is at least about three times or greater then the rate of hydrolysis exhibited by either the first or second polymer component alone, as evaluated using an absorption profiler instrument.

In another form, the composition comprises a two component poly(p-dioxanone) blend having a first polymer component with a weight average molecular weight between about 50,000 to about 100,000 Daltons, and a second polymer component with a weight average molecular weight between about 20,000 to about 30,000 Daltons. The first and second components are blended in ratios with respect to each other of between about 60/40 to 95/5 (weight/weight) percent, respectively. The rate of crystallization of the blended composition is substantially greater than the rate of crystallization exhibited when utilizing either the first or second polymer component alone, when evaluated using isothermal crystallization over a temperature range of between about 40°C to about 80°C. Moreover, the rate of crystallization of the blended composition is at least about three times or greater than the rate of crystallization exhibited when utilizing either the first or second polymer component alone, when evaluated over the temperature range between about 70°C to about 80°C. The blended polymer composition can be crystallized at the isothermal temperature of about 80°C, as verified by means of calorimetric measurements. Similarly, the rate of hydrolysis of the composition, measured in distilled water at a constant pH value is substantially greater then the rate of hydrolysis exhibited by either the first or second polymer component alone, as evaluated using an absorption profiler instrument.

In accordance herewith, a medical device may be produced from a blended absorbable polymeric composition disclosed herein exhibits substantially increased rates of hydrolysis and/or crystallization, as compared to the rate of hydrolysis and/or crystallization of a device produced from an individual polymeric component of the blended composition. The medical devices contemplated herein include those selected from the group consisting of sutures, clips, staples, pins, screws, fibers, stents, gelcaps, tablets, microspheres, meshes, clamps, plates, hooks, buttons, snaps, prosthetics, grafts, injectable polymers, vertebrae discs, anchoring devices, suture anchors, septal occlusion devices, injectable defect fillers, preformed defect fillers, bone waxes, cartilage replacements, spinal fixation devices, drug delivery devices, foams and films.

The blended compositions disclosed herein may further comprise an active medical ingredient substantially homogenously mixed with a polymer or copolymer blend of the present invention. It is envisioned that the active medical ingredient may be released in a living body organism by diffusion and/or a polymer hydrolysis mechanism.

The method of making the bimodal compositions disclosed herein may, in general, comprise a step of blending a first component having a first molecular weight distribution with a second component having a second molecular weight distribution. In one form, the blending step is performed by melting the amounts of first and second components in a sufficient quantity at a temperature above the melting point of the highest melting component, so as to ensure forming a substantially homogenous mixture. In another form, the blending step is performed by dissolving the amounts of first and second molecular weight distributions in a sufficient quantity in a suitable solvent, and subsequently, removing the solvent, thereby forming a substantially homogenous mixture. The dissolving step of the method may further comprise selecting a suitable solvent from the group consisting of acetone, ethyl acetate, ethyl lactide, tetraglycol, chloroform, tetrahydrofuran, dimethyl sulfoxide, N-methyl pyrollidinone, dibutyl phthalate, methylene chloride, methyl ethyl ketone, dibasic esters, methyl isobutyl ketone, dipropylene glycol, dichloromethane and hexafluoroisopropyl alcohol.

Specific embodiments of the present invention will now be described further, by way of example.

### EXAMPLES

Several commercially available instruments were utilized. A description of the equipment used follows.

### Differential Scanning Calorimetry (DSC)

Overall crystallization rates depend principally on two factors: the concentration of growing spherulites over time (nucleation rate) and the rate of spherulitic growth. As expected, these processes have a measurable effect on calorimetric data. Calorimetric results were generated on a TA Instruments Differential Scanning Calorimeter, Model 2910 MDSC, using dry N₂ as a purge gas.

Crystallization studies were conducted in the following manner: after melting, the sample is rapidly cooled to a temperature of interest and the crystallization measured under the controlled isothermal conditions. Isothermal melt crystallizations of the absorbable polymers were conducted as follows: a sample of about 4-5 mg was first melted and maintained for five minutes at temperatures of about 30-40°C above the melting point of the polymer to remove any nucleation sites present in a sample. Test materials were rapidly cooled down (ca. 35°C/min) to the constant test (crystallization) temperature. The isothermal method assumes that no crystallization occurs before the sample reaches the test temperature. In each case, crystallization behavior was characterized over a wide range of temperatures. Calorimetric runs were made in randomized order to avoid any bias due to possible molecular weight degradation. All temperature runs for a given polymer were performed on a single sample. As may be appreciated, the self-consistency of the data engenders confidence that molecular weight loss during testing is not of a concern.

The widely excepted parameter to express the overall crystallization rate is the crystallization half-time, t_{1/2}. This is the time needed for crystallinity to reach 50% conversion.

### Hot-Stage Optical Microscopy (HSOM)

Optical hot stage experiments were conducted using a Mettler FP90 central processor with a Mettler FP82HT hot stage to control sample conditions. The hot stage, with nitrogen flow, was mounted on a Nikon SMZ-U microscope utilizing linear polarized light. The instrument is equipped with IX objective, a set of cross-polarizers and a 1:10 zoom. Images from the microscope were obtained using a Microimage i308 Low Light Integrating Video Camera. The digital images were captured and analyzed using Image Pro Plus (Version 4.0) imaging software.

Growth rate measurements at each temperature were conducted on freshly prepared films to avoid possible degradation problems that might arise with these hydrolytically unstable polyesters. A small amount of ground polymer was placed on the microscope glass slide and a thin cover glass positioned on top of it. The resulting sandwich was then inserted into a hot stage block regulated at temperatures between about 30-40°C above the melting point. The polymeric sample was then melted for five minutes- under a nitrogen purge. A thin film was obtained by applying a slight pressure on the top of cover glass. Monitoring with a digital micrometer, the polymer thickness was adjusted to 0.135inm for each sample run.

### Wide Angle X-ray Diffraction-(WAXD)

Additional supporting evidence was obtained by conventional X-Ray analysis. The WAXD measurements of the isothermally grown films were carried out on a Siemens Hi-Star^{™} unit using CuKα radiation at the wavelength of 1.542 Å. The instrument was operated at 40 kV and 40 mA with the collimator size of ∅ 0.5mm. The convolution of the X-ray images and the calculation of crystallinity content were conducted using DIFFRAC PLUS^{™} software developed by Semens.

### EXAMPLE 1 Crystallization Kinetics of PLLA Homopolymers as a Function of Different Molecular Weights

A series of PLLA homopolymers having substantially different molecular weights were examined calorimetrically. Weight average molecular weights are: 50,000 g/mol (50k), 100,000 (100k), 300,000 (300k), 700,000 (700k). The high molecular weight sample, identified as Test Sample 1, has an inherent viscosity of 7.5 g/dL, but the exact weight average' molecular weight was difficult to determine. Calorimetric evaluation (by DSC) of PLLA samples after quenching from the melt, using the heating rate of 10°C/min revealed glass transition temperatures in the range from 60 (for the lowest molecular weight material) to 64°C (for the highest molecular weight material), and melting temperatures from 177.0 (for the lowest molecular weight material) to 183.5°C (for the highest molecular weight material). Overall crystallinity for PLLA homopolymers, as determined by WAXD, is between 40 and 50%.

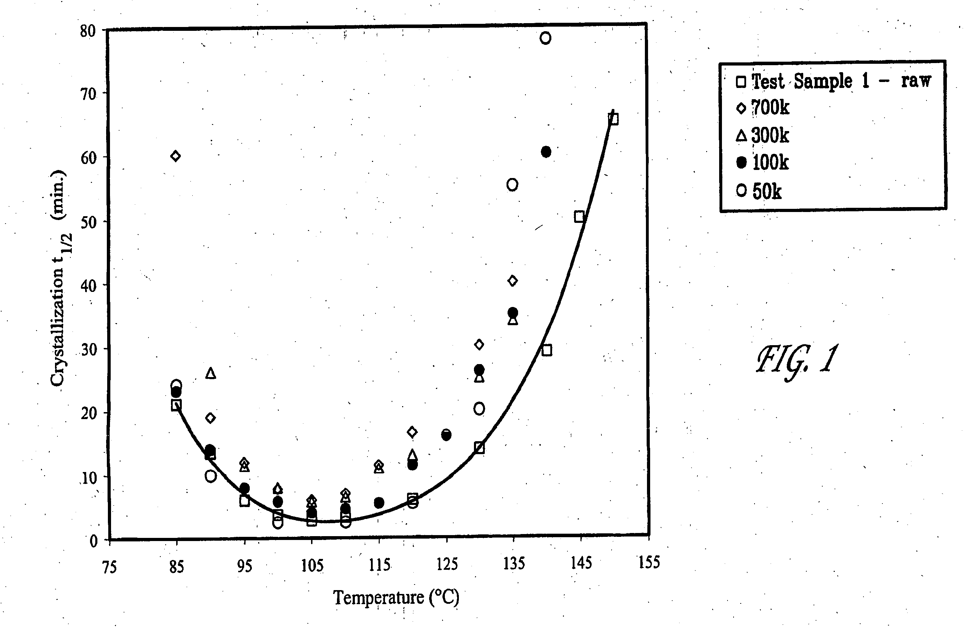

Crystallization properties for the aforementioned polymers were evaluated next under a variety of isothermal conditions utilizing DSC equipment. It was discovered that the high molecular weight polymer used in this study, Test Sample 1, made by solid-state (low temperature) polymerization, exhibited faster crystallization kinetics than other low molecular counterparts. This is demonstrated in FIG. 1, where the crystallization rates (t_{1/2}) for this polymer were compared to the kinetics of the lower molecular weight PLLA samples (50k, 100k, and 300k). It was initially expected that, due to the presence of long, hardly mobile chains of Test Sample 1 polymer, the crystallization rate for this sample would be the slowest at any given Crystallization temperature. Instead, this polymer demonstrated the fastest overall crystallization kinetics, when compared to all samples evaluated. This difference in crystallization rate is exceptionally large in the higher temperature range from 120 to 150°C.

Since crystallization is composed of nucleation and crystal growth, polarized optical microscopy was used to differentiate the contributions of these two processes to the overall crystallization rate. Using HSOM technique it was found that the polymer Test Sample 1 exhibits similar nucleation density when compared with other samples in the PLLA series. However, Test Sample 1 polymer exhibited the fastest spherulitic growth rate, 7.9 µm/min (measured at 130°C), among other counterparts at any given- crystallization temperature. For comparison, a lower molecular weight polymer, 100k was found to have slower spherulitic growth, 6:4 µm/min (at 130°C).

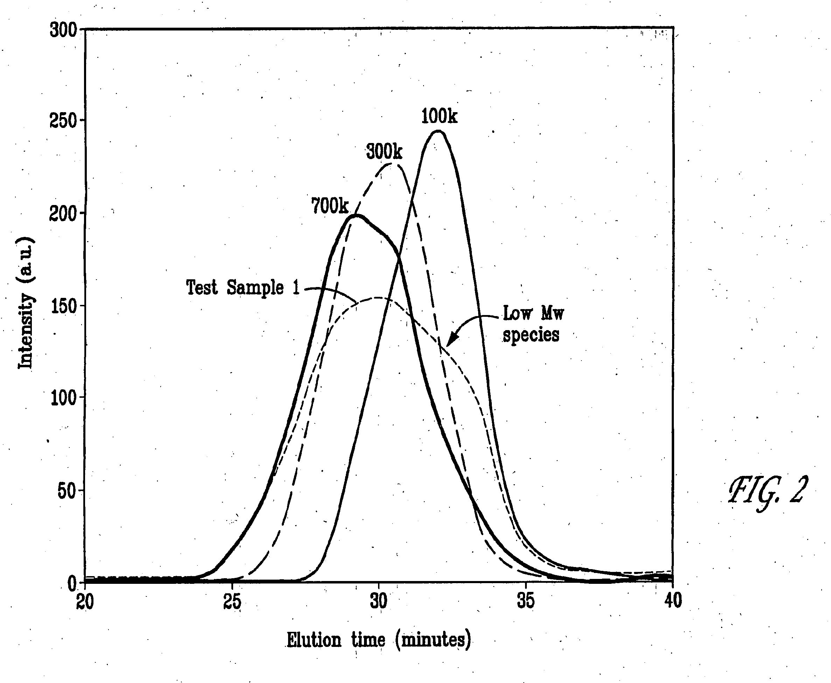

Further investigation of the polymer Test Sample 1 by GPC measurements revealed that this material has a bimodal molecular weight distribution. GPC curves of different PLLA samples-are shown in FIG. 2. As may be seen, the bimodal molecular weight distribution for Test Sample 1 sample is clearly evident. One possible explanation of the bimodal molecular weight distribution in PLLA could be the presence of partial degradation processes generated from the variability in distribution of crystalline and amorphous regions within the sample. These variations might be associated with the inherent nature of the solid-state (low temperature) polymerization used to make this polymer.

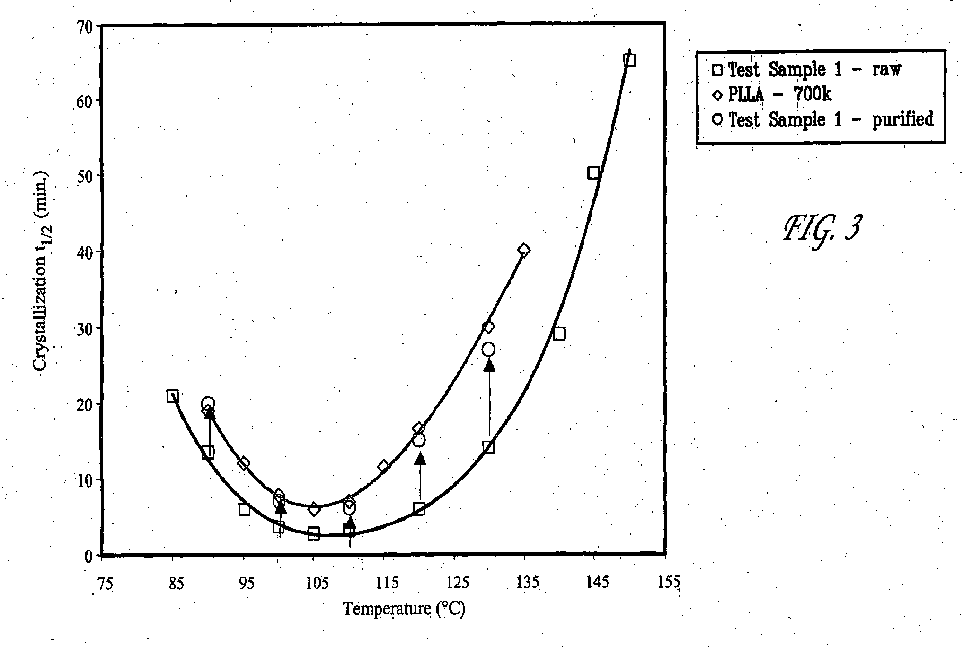

In order to remove the lower molecular weight fraction, the polymer was extracted by acetone. The purified polymer was re-measured by GPC and only a single peak molecular weight distribution was found, confirming the substantial removal of the shorter chain population. The next step was to reexamine the crystallization kinetics of the purified Test Sample 1 sample. It was found that the crystallization rate of the purified Test Sample 1 polymer was considerably reduced, as shown in FIG. 3. At any given crystallization temperature, the fractionated polymer Test Sample 1 exhibits virtually the same crystallization rate as the high molecular weight PLLA-700k polymer. This was a strong indication that the low molecular weight fraction was responsible for the enhanced crystallization kinetics.

### EXAMPLE 2 Crystallization Kinetics of PLLA Blends Exhibiting Enhanced Crystallization Rates

This example presents crystallization results for specifically designed blends of the higher molecular weight PLLA sample (Mw=300,000 g/mol, 300k) and lower molecular weight PLLA (Mw=50,000 g/mol, 50k), in the percent weight ratios of 300k/50k for 80/20, 70/30, and 60/40. In general, lower molecular weight polymers are expected to crystallize faster than their higher molecular weight counterparts due to the higher mobility of their macromolecular chains in the melt phase accompanied by lesser entanglement effects. However, it was discovered that the blending of high molecular weight PLLA with its lower molecular weight counterpart resulted in much higher crystallization rates for the polymer blends examined under a wide range isothermal conditions. At the same time, important physical properties such as the glass transition temperature (T_{g} around 63°C) and the melting point characteristics (Tₘ around 182°C) were found to be in the expected range; these values being directly related to the concentration of individual components in the prepared formulations. As may be appreciated, this feature allows for fine-tuning of desired final mechanical properties of the original higher molecular weight material.

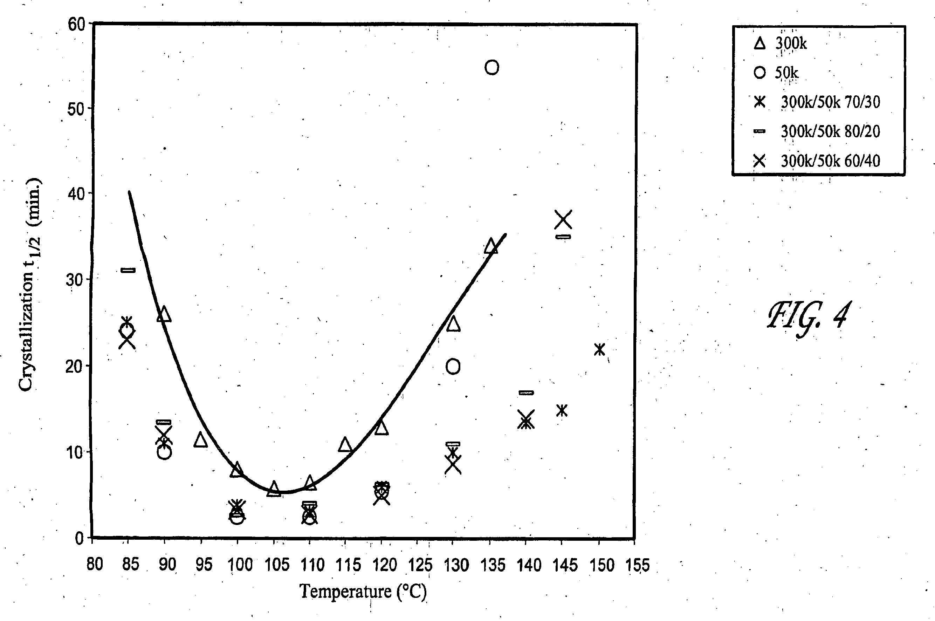

Referring to FIG. 4, blend compositions, of the type disclosed herein, crystallized much faster than the 300k polymer at any given crystallization temperature. At very low temperatures, where the 300k polymer could not readily crystallize (e.g. 85°C or below), it was found that, for all blends, crystallization did occur, demonstrating the strong capability for nucleation and growth. At the intermediate temperature range, using the same set of conditions, blends rich in the 300k polymer exhibited approximately the same crystallization kinetics as a 50k monodisperse polymer, significantly faster than those obtained for the 300k material. However, at temperatures higher than 120°C, data show dramatically faster rates for blends compared to both monodisperse samples (300k & 50k). Intuitively, it would be expected that the data would fall in the range between those generated on neat samples. Furthermore, at temperatures higher than 135°C, two monodisperse samples (300k & 50k) cannot crystallize to the calorimetrically measurable level, while the blends, examined in the same temperature zone, exhibit relatively fast kinetics. The 70/30 blend composition was found to be particularly effective, undergoing measurable crystallization trends even at temperatures as high as 150°C. This may be very useful in certain processing conditions where, for instance, relatively low melt viscosity is required, followed by on line crystallization that can ultimately improve the dimensional stability of the product.

### EXAMPLE 3 Melt index calculation for PLLA polymers at 235°C using standard 3700g weight

Melt index, MI measurements were conducted on 50k and 300k monodisperse samples, as well as on various blends thereof, to investigate the effect of the addition of low molecular weight component on the melt viscosity. These data are presented in Table 1.

**Table 1. Melt index of selected PLLA homopolymers and blends.**

| Polymer | Melt Index, Standard wt. 3,700g | Melt Index, MI (g) wt. 6,600g | Comments |
|---|---|---|---|
| 300k | / | 0.2523 | No flow induced using the standard weight. |
| 50k | / | / | Flow was too fast using the standard weight - not enough material to measure MI. |
| 300k/50k 80/20 | 0.0219 | / | / |
| 300k/50k 70/30 | 0.0374 | / | / |
| 300k/50k 60/40 | 0.0596 | / | / |

| | | | |
|---|---|---|---|
| Instrument: Tinius Olsen Extrusion Plastometer with MP987 Controller, Willow Grove, PA | | | |

Results from Table 1 demonstrate that, in addition to increasing the crystallizability of the polymers, blending improves the melt processability of high molecular weight polymers. The melt index of the blends systematically increased with an increase in the concentration of the low molecular weight component. This finding is very important for the case of a very high molecular weight polymer that may not otherwise be melt processed due to low mobility and high macromolecular chain entanglement.

### EXAMPLE 4 PDS Compositions Having Enhanced Crystallization Rates

In order to demonstrate that the Crystallization kinetics of poly(dioxanone) (PDS) can be improved by the methods disclosed herein, a set of varied weight average molecular weight polymers was employed (80,000g/mol - 80k, and a lower molecular weight counterparts 24,000 g/mol - 24k) to produce an 80k/24k 70/30 wt. % blend. The blends were made by mixing the homopolymers in the melt without using a solvent. Calorimetric data on the monodisperse samples and a blend, subjected to an annealing step at 60°C for 3 hours, using the heating rate of 10°C/min, produced glass transition temperatures of -15.5°C for the 24k polymer, -11.5°C for the 80k polymer, and -12.5°C for the 70/30 blend. Melting points are 103.5°C- for the 24k polymer, 108.5°C for the 80k polymer, and 106.0°C for the 70/30 blend, while overall crystallinity extents are 45% for low molecular weight polymers and 39-40% for regular molecular weight PDS and their 70/30 blend.

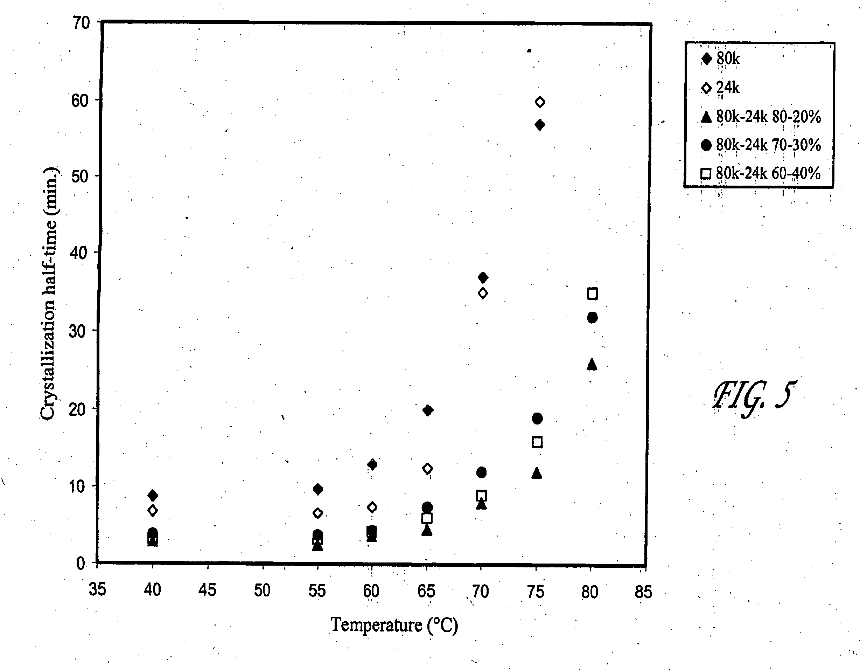

Isothermal crystallization measurements were performed next, using the DSC crystallization procedure described earlier in the text. As shown in FIG. 5 similar to PLLA case; it was found that 60/40, 70/30 and 80/20 PDS blends crystallize significantly faster than the monodisperse PDS components (80k and 24k) alone. Crystallization rates dramatically improved in each temperature regime studied. Furthermore, at the highest temperature zone (80°C), both monodisperse samples showed no crystallization pattern by DSC; on the other hand, using the same conditions, crystallization was detected and the rate was calculated for 60/40, 70/30 and 80/20 PDS blend.

### EXAMPLE 5 Effect of Different PDS Blend Compositions on Isothermal and Non-isothermal Crystallization Rates

This example presents crystallization and mechanical properties of PDS homopolymers 80k and 24k, and their 95/5 and 90/10 blends. The glass transition temperature and melting characteristics of the 95/5 and 90/10 blends are nearly identical to the 80k PDS reported earlier in the text.

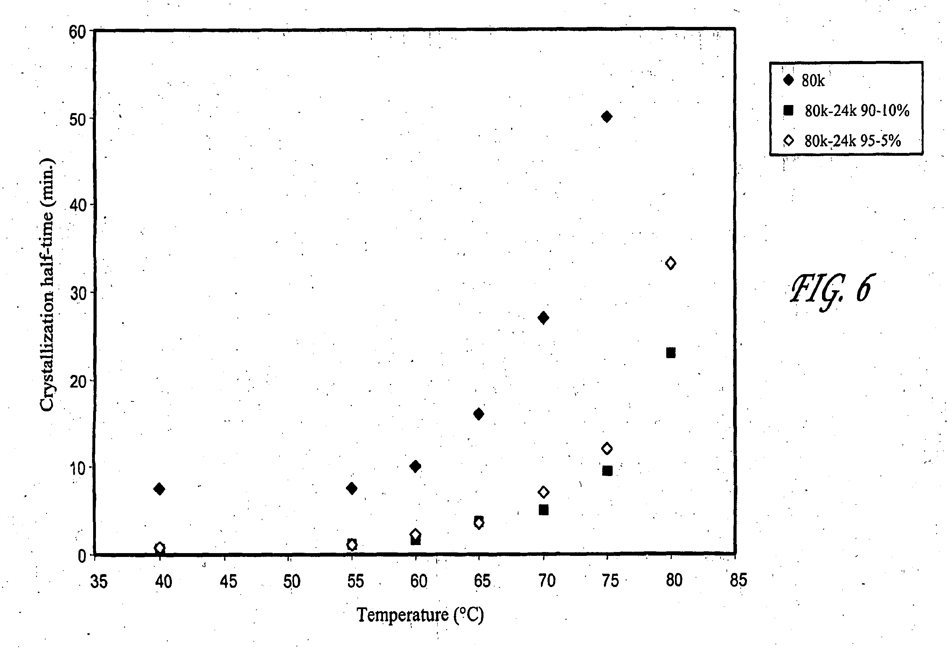

Isothermal crystallization data of these blend formulations were examined first. Again, the data showed a dramatic increase in crystallization rates for the two blends, as compared with PDS having regular and lower molecular weight chains. FIG. 6 presents results obtained for an 80k PDS polymer and two of blends containing a very low concentration of a 24k PDS polymer component. Only 5% of low molecular weight polymer appears to be necessary to produce a beneficial effect on the crystallization rate of a PDS homopolymer. This would be expected to be very important in fiber extrusion applications where the mechanical strength associated with higher molecular weight material must be preserved.

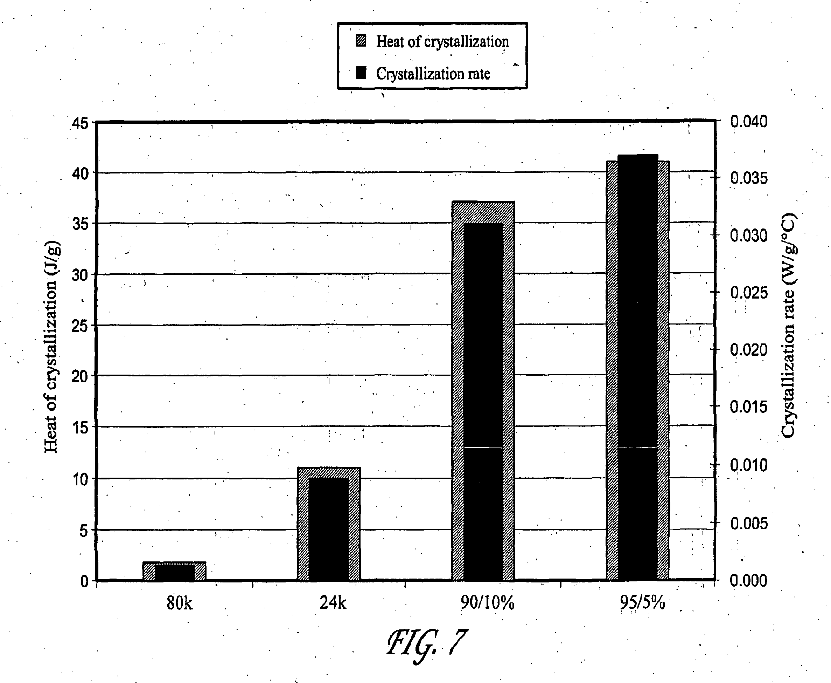

Non-isothermal DSC crystallization data were obtained for several PDS polymers during cooling from the melt at a constant cooling rate of 10°C/min. Again, a dramatic increase in crystallization rates was observed for the two blends compared to both PDS homopolymers using this non-isothermal method. As shown in FIG. 7, values for the enthalpy (heat) of crystallization, ΔHc, developed during the cooling step, as well as the crystallization rate (obtained from the initial slope of the crystallization peak) for 90/10 and 95/5 blends are higher than the corresponding values for 80k and 24k PDS homopolymers.

### EXAMPLE 6 Effect of Different PDS Blend Compositions on Mechanical Tensile-Strength Properties

Selected mechanical properties of regular molecular weight PDS film and three blends 95/5, 90/10, and 80/20 were examined using an Instron Tensile testing machine to determine the effect of the addition of low molecular weight PDS component on the mechanical properties of films prepared from studied materials. These data are summarized in Table 2, below.

**Table 2. Selected mechanical properties of PDS films.**

| **Polymer** | **Load at Peak (lbf)** | **% Strain at Peak** | **Displacement at break (in)** | **Young's Modulus (ksi)** |
|---|---|---|---|---|
| **Regular PDS, 80k** | 14.0 | 1056 | 5.3 | 48 |
| **80/20 blend** | 12.5 | 1250 | 6.3 | 43 |
| **90/10 blend** | 13.0 | 1211 | 6.1 | 40 |
| **95/5 blend** | 13.5 | 1125 | 5.7 | 45 |

The mechanical results in Table 2 suggest that only a minimal effect on the mechanical properties for the three blends was detected when compared to the properties of the 80k unblended material. Moreover, in the case of the 95/5 blend, the effect was substantially negligible. This represents an important discovery that suggests that the blending of relatively small amount of lower molecular weight polymer does not diminish the final physical properties of a product.

### EXAMPLE 7 Enhanced Absorption rates for PLLA Blends

The hydrolysis profile method determines the hydrolytic degradation time of ester-containing samples. The hydrolysis profile is generated by first hydrolytically degrading a test specimen, while maintaining a constant pH by titrating with a standard base and measuring the quantity of base used with time. This measurement and titration procedure is automated through the use of a pH stat instrument (718 STAT Titrator Complete, by MetroOhm, using Software TiNet 2.4). The samples are placed in a 70mL stirred, sealed, bath of deionized water held at 75°C+/-0:2°C and at a pH of 7.27 Each sample bath is continuously monitored for pH changes (drops in pH) from the set point of 7.27. If any decrease is measured, a sodium hydroxide solution is added to return to the bath 7.27 (NaOH 0.05N). The following measurements are recorded by computer: temperature, volume of base added (V(t)), and pH, over time. The V(t) time-course is analyzed to yield the time to 50% hydrolysis, t 50. Prior to each sample run, the pH probe at each test station is calibrated at pH values of 4.0, 7.0 and 10.0, using standard solutions.

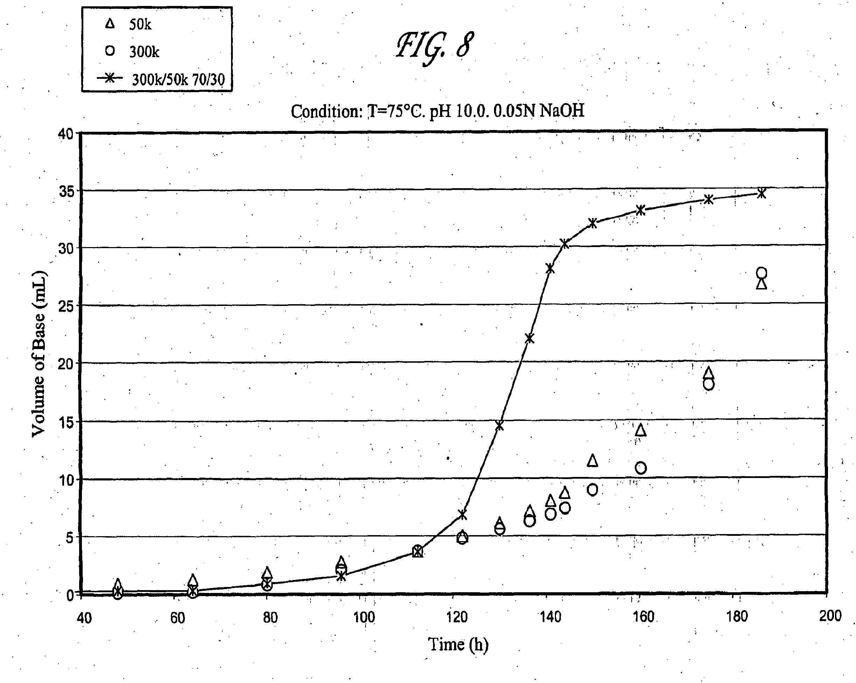

Hydrolysis measurements of PLLA samples were conducted using the automated hydrolysis profile at 75°C, a constant pH value of 10.0 and a sodium hydroxide solution (0.05N) as a base. Prior to the experiments all samples were annealed using the same temperature condition, namely, 100°C for 12 hours. Hydrolysis data obtained on solvent cast films made from PLLA homopolymers, 300k, 50k and their 70/30 wt.% blend are shown in FIG. 8. A faster absorption rate for the 70/30 blend was observed over the individual components alone. A direct comparison of kinetic parameters, including comparing the time at which 50% of the polymer hydrolyzed, suggests that the hydrolysis rate for the 70/30 blend was three or more times faster than that observed for the individual components, alone. It would be expected that the hydrolysis rate value of the blend should reside in between the rates observed for the 300k and 50k samples. This, in addition to the potential uses in the medical device and drug delivery sectors, may additionally provide a beneficial impact on the waste disposal issues for PLLA based polymers when used as packaging materials.

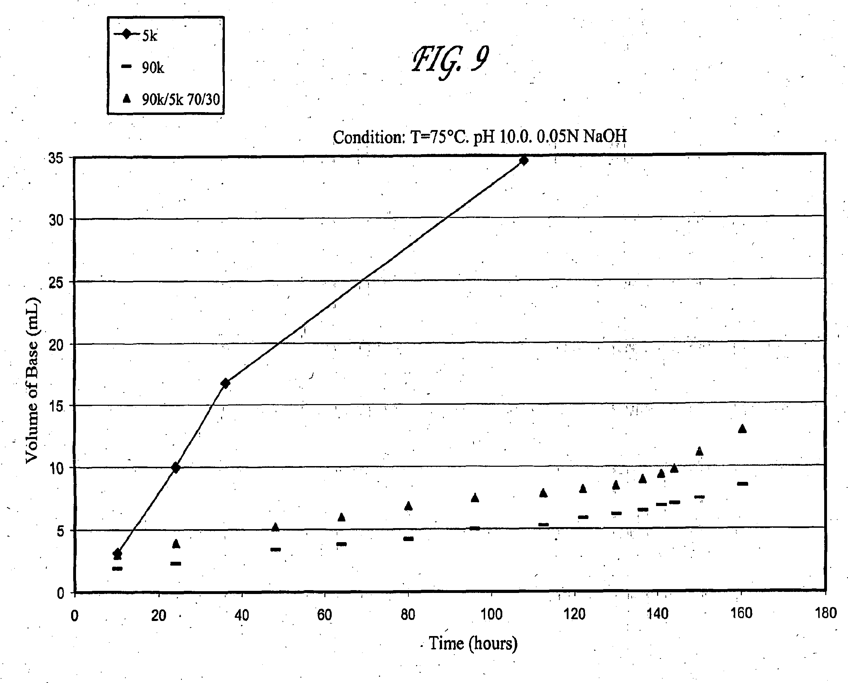

For comparative purposes, FIG. 9 presents the hydrolysis profiles obtained for solvent cast films made from both 5,000 Dalton and 90,000 Dalton PLEA homopolymers, and a 70/30 wt.% blend of 90,000 to 5,000 homopolymer. As may be appreciated, the 5,000 Dalton PLLA homopolymer is well below the weight average molecular weight range of about 20,000. to about 50,000 Daltons and, as would be expected, the data presented in FIG. 9 fails to exhibit the benefits of the blends produced in accordance herewith.

### EXAMPLE 8 Enhanced Absorption Rates of Various PDS Polymers and Blends

Example 8 examines a wider range of PDS blend compositions, including 95/5, 90/10, 80/20 and 70/30 blends.

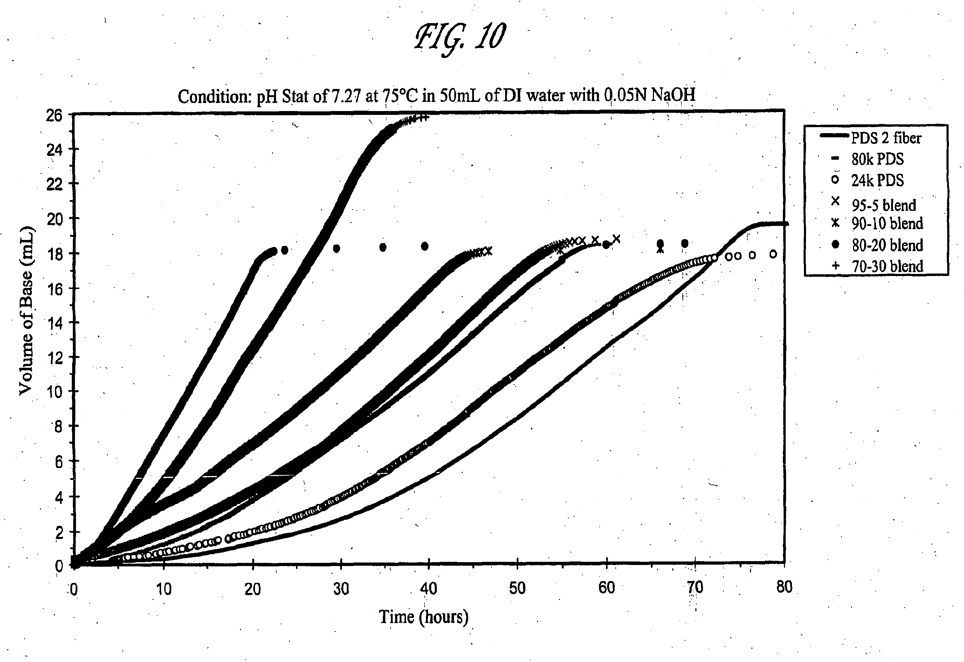

Hydrolysis measurements of PDS samples were carried on using the automates hydrolysis profile at 75°C, at a constant pH value of 7.27 (neutral) and a sodium hydroxide solution (0.05N) as a base. Prior to the experiments, all compression molded films were annealed using the same temperature condition, 70°C for 10 minutes. Hydrolysis data were obtained for 80k, and 24k PDS homopolymers, as well as for various blends thereof. Data are presented in FIG. 10.

Again, considerably faster absorption profiles for all PDS blends were observed when compared to the monodisperse samples, with the 80/20 blend showing the greatest effect. PDS monofilament, on the other hand, exhibited the slowest hydrolysis rate, due to the high molecular orientation of both crystalline and amorphous chains, which makes water diffusion particularly more difficult.

## Claims

1. A bimodal polymer composition, comprising:
(a) a first amount of a bioabsorbable polymer having a first molecular weight distribution; and
(b) a second amount of said bioabsorbable polymer having a second molecular weight distribution having a weight average molecular weight between 20,000 to 50,000 Daltons, the weight average molecular weight ratio of said- -first molecular weight distribution to said second molecular weight distribution is at least two to one;
wherein a substantially homogeneous blend of said first and second amounts of said bioabsorbable polymer is formed in a ratio of between 50/50 to 95/5 weight/weight percent.

2. The bimodal polymer composition of claim 1, wherein said bioabsorbable polymer is semi-crystalline.

3. The bimodal polymer composition of claim 2; having a degree of crystallinity from 10% to 50%.

4. The bimodal polymer composition of claim 1, wherein the bioabsorbable polymer is selected from the group consisting of poly(lactide); poly(glycolide), poly(dioxanone), poly(ε-caprolactone), poly(hydroxybutyrate), poly(β-hydroxybutyrate), poly(hydroxyvalerate), poly(tetramethylene carbonate), poly(amino acids) and copolymers and terpolymers thereof.

5. The bimodal polymer composition of claim 1, wherein said first molecular weight distribution is a weight average molecular weight from between 50,000 to 2,000,000 Daltons.

6. A medical device produced from a process comprising (i) the step of injection molding or extruding the medical device from a bimodal polymer composition or (ii) the step of subjecting a medical device made from said bimodal polymer composition to a heat treatment step, said bimodal polymer composition comprising:
(a) a first amount of a bioabsorbable polymer polymerized so as to have a first molecular weight distribution; and
(b) a second amount of said bioabsorbable polymer polymerized so as to have a second molecular weight distribution having a weight average molecular weight between 20,000 to 50,000 Daltons, the weight average molecular weight ratio of said first molecular weight distribution to said second molecular weight distribution is at least two to one; wherein a substantially homogeneous blend of said first and second amounts of said bioabsorbable polymer is formed in a ratio of between 50/50 to 95/5 weight/weight percent.

7. A medical device according to claim 6, wherein:
said bioabsorbable polymer is a polylactide polymer;
said first molecular weight distribution is between 100,000 to 1,000,000 Daltons;
said substantially homogeneous blend of said first and second amounts of said bioabsorbable polymer is formed in a ratio of between 60/40 to 80/20 weight/weight percent; and
said heat treatment step is over a temperature range of between 85°C to 150°C.

8. A medical device according to claim 6, wherein:
said bioabsorbable polymer is a poly(dioxanone) polymer;
said first molecular weight distribution is between 50,000 to 100,000 Daltons;
said substantially homogeneous blend of said first and second amounts of said bioabsorbable polymer is formed in a ratio of between 60/40 to 95/5 weight/weight percent; and
said heat treatment step is over a temperature range of between 40°C to 80°C.

9. The medical device of claim 6, 7 or 8, wherein the medical device is selected from the group consisting of a suture, a clip, a staple, a pin, a screw, a fiber, a mesh, a clamp, a plate, a hook, a button, a snap, a prosthetic, a graft, an injectable polymer, a vertebrae disc, an anchoring device, a suture anchor, a septal occlusion device, an injectable defect filler, a preformed defect filler, a bone wax, a cartilage replacement, a spinal fixation device, a drug delivery device, a foam and a film.

10. A method of making a medical device, comprising (i) the step of injection molding or extruding the medical device from a bimodal polymer composition or (ii) the step of subjecting a medical device made from the bimodal polymer composition to a heat treatment step, the polymer composition, comprising:
(a) a first amount of a bioabsorbable polymer polymerized so as to have a first molecular weight distribution; and
(b) a second amount of the bioabsorbable polymer polymerize so as to have a second molecular weight distribution having a weight average molecular weight between 20,000 to 50,000 Daltons, the weight average molecular weight ratio of the first molecular weight distribution to the second molecular weight distribution is at least two to one;
wherein a substantially homogeneous blend of said first and second amounts of said bioabsorbable polymer is formed in a ratio of between 60/40 to 95/5 weight/weight percent.

11. A method of making a medical device according to claim 10, wherein:
said bioabsorbable polymer is a polylactide polymer;
said first molecular weight distribution is between 100,000 to 1,000,000 Daltons;
said substantially homogeneous blend of said first and second amounts of said bioabsorbable polymer is formed in a ratio of between- 60/40 to 80/20 weight/weight percent; and
said heat treatment step is over a temperature range of between 85°C to 150°C.

12. The method according to claim 11, where the first amount of a poly(L-lactide) polymer has a first molecular weight distribution between 100,000 to 500,000 Daltons.

13. The method according to claim 11, where the temperature range is between 140°C to 150°C.

14. A method of making a medical device according to claim 10, wherein:
said bioabsorbable polymer is a poly(dioxanone) polymer;
said first molecular weight distribution is between 50,000 to 100,000 Daltons;
said substantially homogeneous blend of said first and second amounts of said bioabsorbable polymer is formed in a ratio of between 60/40 to 95/5 weight/weight percent; and
said heat treatment step is over a temperature range of between 40°C to 80°C.

15. The method according to claim 14, where the temperature range is between 70°C to 80°C.

16. The method according to any of claims 10 to 15, wherein the blend is produced using a melt blending step.

17. The method according to any of claims 10 to 15, wherein the blend is produced in the presence of a solvent.

18. The method according to claim 17, wherein the solvent is selected from the group consisting of acetone, ethyl acetate, ethyl lactate, tetraethyleneglycol, chloroform, tetrahydrofuran, dimethyl sulfoxide, 1-methyl-2-pyrollidinone, dibutyl phthalate; methylethyl ketone, dibasic esters; methyl isobutyl ketone, dipropylene glycol, dichloromethane and hexafluoroisopropyl alcohol.

## Patentansprüche

1. Bimodale Polymerzusammensetzung, umfassend:
(a) eine erste Menge eines bioabsorbierbaren Polymers mit einer ersten Molekulargewichtsverteilung; und
(b) eine zweite Menge des bioabsorbierbaren Polymers mit einer zweiten Molekulargewichtsverteilung mit einem Molekulargewicht im Gewichtsmittel zwischen 20.000 bis 50.000 Daltons, wobei das Molekulargewichtsverhältnis im Gewichtsmittel der ersten Molekulargewichtsverteilung zur zweiten Molekulargewichtsverteilung wenigstens zwei zu eins beträgt;
wobei ein im wesentlichen homogenes Gemisch aus den ersten und zweiten Mengen des bioabsorbierbaren Polymers in einem Verhältnis zwischen 50/50 bis 95/5 Gewicht/Gewicht-Prozent gebildet ist.

2. Bimodale Polymerzusammensetzung nach Anspruch 1, wobei das bioabsorbierbare Polymer halbkristallin ist.

3. Bimodale Polymerzusammensetzung nach Anspruch 2, die einen Kristallinitätsgrad von 10% bis 50% aufweist.

4. Bimodale Polymerzusammensetzung nach Anspruch 1, wobei das bioabsorbierbare Polymer ausgewählt ist aus der Gruppe, bestehend aus Poly(lactid), Poly(glykolid), Poly(dioxanon), Poly(ε-caprolacton), Poly(hydroxybutyrat), Poly(β-hydroxybutyrat), Poly(hydroxyvalerat), Poly(tetramethylencarbonat), Poly(aminosäuren) und Copolymeren und Terpolymeren davon.

5. Bimodale Polymerzusammensetzung nach Anspruch 1, wobei die erste Molekulargewichtsverteilung ein Molekulargewicht im Gewichtsmittel von zwischen 50.000 bis 2.000.000 Daltons ist.

6. Medizinische Vorrichtung, hergestellt aus einem Verfahren, das (i) den Schritt des Spritzgießens oder Extrudierens der medizinischen Vorrichtung aus einer bimodalen Polymerzusammensetzung oder (ii) den Schritt des Unterwerfens einer medizinischen Vorrichtung, die hergestellt ist aus der bimodalen Polymerzusammensetzung, unter einen Wärmebehandlungsschritt umfasst, wobei die bimodale Polymerzusammensetzung:
(a) eine erste Menge eines bioabsorbierbaren Polymers, die so polymerisiert ist, dass sie eine erste Molekulargewichtsverteilung aufweist; und
(b) eine zweite Menge des bioabsorbierbaren Polymers, die so polymerisiert ist, dass sie eine zweite Molekulargewichtsverteilung aufweist, mit einem Molekulargewicht im Gewichtsmittel zwischen 20.000 bis 50.000 Daltons, wobei das Molekulargewichtsverhältnis im Gewichtsmittel der ersten Molekulargewichtsverteilung zur zweiten Molekulargewichtsverteilung wenigstens zwei zu eins beträgt;
wobei ein im wesentlichen homogenes Gemisch aus den ersten und zweiten Mengen des bioabsorbierbaren Polymers in einem Verhältnis zwischen 50/50 bis 95/5 Gewicht/Gewicht-Prozent gebildet ist. umfasst.

7. Medizinische Vorrichtung nach Anspruch 6, wobei:
das bioabsorbierbare Polymer ein Polylactid-Polymer ist;
die erste Molekulargewichtsverteilung zwischen 100.000 bis 1.000.000 Daltons liegt;
das im wesentlichen homogene Gemisch aus den ersten und zweiten Mengen des bioabsorbierbaren Polymers in einem Verhältnis von zwischen 60/40 bis 80/20 Gewicht/Gewicht-Prozent gebildet ist; und
der Wärmebehandlungsschritt über einen Temperaturbereich von zwischen 85°C bis 150°C ist.

8. Medizinische Vorrichtung nach Anspruch 6, wobei:
das bioabsorbierbare Polymer ein Poly(dioxanon)-Polymer ist;
die erste Molekulargewichtsverteilung zwischen 50.000 bis 100.000 liegt;
das im wesentlichen homogene Gemisch aus den ersten und zweiten Mengen des bioabsorbierbaren Polymers in einem Verhältnis von zwischen 60/40 bis 95/5 Gewicht/Gewicht-Prozent gebildet ist; und
der Wärmebehandlungsschritt über einen Temperaturbereich von zwischen 40°C bis 80°C ist.

9. Medizinische Vorrichtung nach Anspruch 6, 7 oder 8, wobei die medizinische Vorrichtung ausgewählt ist aus der Gruppe, bestehend aus einem Nahtmaterial, einem Clip, einer Klammer, einem Stift, einer Schraube, einer Faser, einem Netz, einer Klemme, einer Platte, einem Haken, einem Knopf, einem Schnappverschluss, einer Prothese, einem Transplantat, einem injizierbaren Polymer, einer Bandscheibe, einer Verankerungsvorrichtung, einem Nahtmaterialanker, einer Septumokklusionsvorrichtung, einem injizierbaren Defektfüllstoff, einem vorgeformten Defektfüllstoff, einem Knochenwachs, einem Knorpelersatzstoff, einer Wirbelsäulenfixierungsvorrichtung, einer Arzneistoffabgabevorrichtung, einem Schaum und einem Film.

10. Verfahren zur Herstellung einer medizinischen Vorrichtung, das (i) den Schritt des Spritzgießens oder Extrudierens der medizinischen Vorrichtung aus einer bimodalen Polymerzusammensetzung oder (ii) den Schritt des Unterwerfens einer medizinischen Vorrichtung, die hergestellt ist aus der bimodalen Polymerzusammensetzung, unter einen Wärmebehandlungsschritt umfasst, wobei die Polymerzusammensetzung:
(a) eine erste Menge eines bioabsorbierbaren Polymers, die so polymerisiert ist, dass sie eine erste Molekulargewichtsverteilung aufweist; und
(b) eine zweite Menge des bioabsorbierbaren Polymers, die so polymerisiert ist, dass sie eine zweite Molekulargewichtsverteilung aufweist, mit einem Molekulargewicht im Gewichtsmittel zwischen 20.000 bis 50.000 Daltons,
wobei das Molekulargewichtsverhältnis im Gewichtsmittel der ersten Molekulargewichtsverteilung zur zweiten Molekulargewichtsverteilung wenigstens zwei zu eins beträgt;
wobei ein im wesentlichen homogenes Gemisch der ersten und zweiten Mengen des bioabsorbierbaren Polymers in einem Verhältnis von zwischen 60/40 bis 95/5 Gewicht/Gewicht-Prozent gebildet ist,
umfasst.

11. Verfahren zur Herstellung einer medizinischen Vorrichtung nach Anspruch 10, wobei:
das bioabsorbierbare Polymer ein Polylactid-Polymer ist;
die erste Molekulargewichtsverteilung zwischen 100.000 bis 1.000.000 liegt;
das im wesentlichen homogene Gemisch der ersten und zweiten Mengen des bioabsorbierbaren Polymers in einem Verhältnis zwischen 60/40 bis 80/20 Gewicht/Gewicht-Prozent gebildet ist; und
der Wärmebehandlungsschritt im Temperaturbereich von zwischen 85°C bis 150°C ist.

12. Verfahren nach Anspruch 11, wobei die erste Menge eines Poly(L-lactid)-Polymers eine erste Molekulargewichtsverteilung zwischen 100.000 bis 500.000 Daltons aufweist.

13. Verfahren nach Anspruch 11, wobei der Temperaturbereich zwischen 140°C bis 150°C liegt.

14. Verfahren zur Herstellung einer medizinischen Vorrichtung nach Anspruch 10, wobei:
das bioabsorbierbare Polymer ein Poly(dioxanon)-Polymer ist;
die erste Molekulargewichtsverteilung zwischen 50.000 bis 100.000 Daltons liegt;
das im wesentlichen homogene Gemisch aus den ersten und zweiten Mengen des bioabsorbierbaren Polymers in einem Verhältnis von zwischen 60/40 bis 95/5 Gewicht/Gewicht-Prozent gebildet ist; und
der Wärmebehandlungsschritt über einen Temperaturbereich von zwischen 40°C bis 80°C ist.

15. Verfahren nach Anspruch 14, wobei der Temperaturbereich zwischen 70°C bis 80°C liegt.

16. Verfahren nach einem der Ansprüche 10 bis 15, wobei das Gemisch unter Verwendung eines Schmelzvermischungsschrittes hergestellt wird.

17. Verfahren nach einem der Ansprüche 10 bis 15, wobei das Gemisch in Gegenwart eines Lösemittels hergestellt wird.

18. Verfahren nach Anspruch 17, wobei das Lösemittel ausgewählt wird aus der Gruppe, bestehend aus Aceton, Ethylacetat, Ethyllactat, Tetraethylenglykol, Chloroform, Tetrahydrofuran, Dimethylsulfoxid, 1-Methyl-2-pyrollidinon, Dibutylphthalat, Methylethylketon, zweibasigen Estern, Methylisobutylketon, Dipropylenglykol, Dichlormethan und Hexafluorisopropylalkohol.

## Revendications

1. Composition polymère bimodale comprenant :
(a) une première quantité d'un polymère bioabsorbable ayant une première distribution de poids moléculaire ; et
(b) une seconde quantité dudit polymère bioabsorbable ayant une seconde distribution de poids moléculaire ayant un poids moléculaire moyen en poids compris dans la plage allant de 20 000 à 50 000 Daltons, le rapport en poids moléculaire moyen en poids de ladite première distribution de poids moléculaire à ladite seconde distribution de poids moléculaire est d'au moins 2 pour 1 ;
dans laquelle un mélange essentiellement homogène desdites première et seconde quantités dudit polymère bioabsorbable est formé dans un rapport compris dans la plage allant de 50/50 à 95/5 pourcent en poids/poids.

2. Composition polymère bimodale selon la revendication 1, dans laquelle ledit polymère bioabsorbable est semi-cristallin.

3. Composition polymère bimodale selon la revendication 2, ayant un degré de cristallinité de 10 % à 50 %.

4. Composition polymère bimodale selon la revendication 1, dans laquelle le polymère bioabsorbable est choisi dans le groupe constitué de poly(lactide), poly(glycolide), poly-(dioxanone), poly(ε-caprolactone), poly(hydroxybutyrate), poly(β-hydroxybutyrate), poly(hydroxyvalérate), poly(carbonate de tétraméthylène), poly(acides aminés) et copolymères et terpolymères de ceux-ci.

5. Composition polymère bimodale selon la revendication 1, dans laquelle ladite première distribution de poids moléculaire est un poids moléculaire moyen en poids compris dans la plage allant de 50 000 à 2 000 000 Daltons.

6. Dispositif médical produit grâce à un procédé comprenant (i) l'étape de moulage ou d'extrusion par injection du dispositif médical à partir d'une composition polymère bimodale ou (ii) l'étape de soumission d'un dispositif médical préparé à partir de ladite composition polymère bimodale à une étape de traitement thermique, ladite composition polymère bimodale comprenant :
(a) une première quantité d'un polymère bioabsorbable polymérisé de sorte à avoir une première distribution de poids moléculaire ; et
(b) une seconde quantité dudit polymère bioabsorbable polymérisé de sorte à avoir une seconde distribution de poids moléculaire ayant un poids moléculaire moyen en poids compris dans la plage allant de 20 000 à 50 000 Daltons, le rapport en poids moléculaire moyen en poids de ladite première distribution de poids moléculaire à ladite seconde distribution de poids moléculaire est d'au moins 2 pour 1 ;
dans laquelle un mélange essentiellement homogène desdites première et seconde quantités dudit polymère bioabsorbable est formé dans un rapport compris dans la plage allant de 50/50 à 95/5 pourcent en poids/poids.

7. Dispositif médical selon la revendication 6, dans lequel
· ledit polymère bioabsorbable est un polymère poly(lactide) ;
· ladite première distribution de poids moléculaire est comprise dans la plage allant de 100 000 à 1 000 000 Daltons ;
· ledit mélange essentiellement homogène desdites première et seconde quantités dudit polymère bioabsorbable est formé dans un rapport compris dans la plage allant de 60/40 à 80/20 pourcent en poids/poids ; et
· ladite étape de traitement thermique est réalisée sur une plage de température allant de 85°C à 150°C.

8. Dispositif médical selon la revendication 6, dans lequel
· ledit polymère bioabsorbable est un polymère poly(dioxanone) ;
· ladite première distribution de poids moléculaire est comprise dans la plage allant de 50 000 à 100 000 Daltons ;
· ledit mélange essentiellement homogène desdites première et seconde quantités dudit polymère bioabsorbable est formé dans un rapport compris dans la plage allant de 60/40 à 95/5 pourcent en poids/poids ; et
· ladite étape de traitement thermique est réalisée sur une plage de température allant de 40°C à 80°C.

9. Dispositif médical selon la revendication 6, 7 ou 8, dans lequel le dispositif médical est choisi dans le groupe constitué par une suture, une pince, une agrafe, une épingle, une vis, une fibre, une maille, une clamp, une plaque, un crochet, un bouton, un mousqueton, une prothèse, une greffe, un polymère injectable, un disque vertébral, un dispositif d'ancrage, une ancre de suture, un dispositif d'occlusion septale, un matériau de comblement injectable, un matériau de comblement préformé, une cire osseuse, un remplacement de cartilage, un dispositif de fixation spinale, un dispositif de délivrance de médicament, une mousse et un film.

10. Procédé de préparation d'un dispositif médical, comprenant (i) l'étape de moulage ou d'extrusion par injection du dispositif médical à partir d'une composition polymère bimodale ou (ii) l'étape de soumission d'un dispositif médical préparé à partir de ladite composition polymère bimodale à une étape de traitement thermique, ladite composition polymère bimodale comprenant :
(a) une première quantité d'un polymère bioabsorbable polymérisé de sorte à avoir une première distribution de poids moléculaire ; et
(b) une seconde quantité dudit polymère bioabsorbable polymérisé de sorte à avoir une seconde distribution de poids moléculaire ayant un poids moléculaire moyen en poids compris dans la plage allant de 20 000 à 50 000 Daltons, le rapport en poids moléculaire moyen en poids de ladite première distribution de poids moléculaire à ladite seconde distribution de poids moléculaire est d'au moins 2 pour 1 ;
dans laquelle un mélange essentiellement homogène desdites première et seconde quantités dudit polymère bioabsorbable est formé dans un rapport compris dans la plage allant de 60/40 à 95/5 pourcent en poids/poids.

11. Procédé de préparation d'un dispositif médical selon la revendication 10, dans lequel
· ledit polymère bioabsorbable est un polymère poly(lactide) ;
· ladite première distribution de poids moléculaire est comprise dans la plage allant de 100 000 à 1 000 000 Daltons ;
· ledit mélange essentiellement homogène desdites première et seconde quantités dudit polymère bioabsorbable est formé dans un rapport compris dans la plage allant de 60/40 à 80/20 pourcent en poids/poids ; et
· ladite étape de traitement thermique est réalisée sur une plage de température allant de 85°C à 150°C.

12. Procédé selon la revendication 11, dans lequel la première quantité d'un polymère poly(L-lactide) a une première distribution de poids moléculaire comprise dans la plage allant de 100 000 à 500 000 Daltons.

13. Procédé selon la revendication 11, dans lequel la plage de température est entre 140°C à 150°C.

14. Procédé de préparation d'un dispositif médical selon la revendication 10, dans lequel
· ledit polymère bioabsorbable est un polymère poly(dioxanone) ;
· ladite première distribution de poids moléculaire est comprise dans la plage allant de 50 000 à 100 000 Daltons ;
· ledit mélange essentiellement homogène desdites première et seconde quantités dudit polymère bioabsorbable est formé dans un rapport compris dans la plage allant de 60/40 à 95/5 pourcent en poids/poids ; et
· ladite étape de traitement thermique est réalisée sur une plage de température allant de 40°C à 80°C.

15. Procédé selon la revendication 14, dans lequel la plage de température est entre 70°C à 80°C.

16. Procédé selon l'une quelconque des revendications 10 à 15, dans lequel le mélange est produit en utilisant une étape de mélange à l'état fondu.

17. Procédé selon l'une quelconque des revendications 10 à 15, dans lequel le mélange est produit en présence d'un solvant.

18. Procédé selon la revendication 17, dans lequel le solvant est choisi dans le groupe constitué d'acétone, acétate d'éthyle, lactate d'éthyle, tétraéthylène glycol, chloroforme, tétrahydrofurane, diméthylsulfoxyde, 1-méthyl-2-pyrrolidinone, phtalate de dibutyle, méthyléthylcétone, esters dibasiques, méthylisobutylcétone, dipropylène glycol, dichlorométhane et d'alcool hexafluoroisopropylique.
